# EUROPEAN PATENT SPECIFICATION

(11) **EP 4 063 497 B1**
(45) Date of publication and mention of the grant of the patent: **24.04.2024**
(21) Application number: 21870540.8
(22) Date of filing: 03.12.2021
(51) Int. Cl.: C12N 5/20, C07K 16/40, C12N 15/13, G01N 33/573, G01N 33/577, C07K 16/12

(54) **ANTI-IMP TYPE CARBAPENEMASE HYBRIDOMA CELL STRAIN, MONOCLONAL ANTIBODY AND USE**
CARBAPENEMASE-RESISTENTE HYBRIDOMA-ZELLINIE VOM IMP-TYP SOWIE MONOKLONALER ANTIKÖRPER (MAB) UND VERWENDUNG DAVON
SOUCHE CELLULAIRE D'HYBRIDOME DE CARBAPENEMASE DE TYPE ANTI-IMP, ANTICORPS MONOCLONAL ET UTILISATION

(30) Priority: 07.02.2021 CN 202110166074
(43) Date of publication of application: 28.09.2022
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN)
(72) Inventor: YUAN, Qinghua, Tianjin 300480 (CN); HE, Yongsheng, Tianjin 300480 (CN); LI, Keke, Tianjin 300480 (CN); CHEN, Xiaoling, Tianjin 300480 (CN); ZHOU, Yuehui, Tianjin 300480 (CN); FAN, Linlin, Tianjin 300480 (CN); WANG, Yufang, Tianjin 300480 (CN); ZANG, Danrong, Tianjin 300480 (CN)
(74) Representative: Bayramoglu et al.
(86) International application number: PCT/CN2021/135403
(87) International publication number: WO 2022/166353

(56) References cited:
- EP-A1- 3 230 741
- WO-A1-2016/092096
- CN-A- 107 667 291
- CN-A- 112 500 489
- CN-A- 112 501 131
- US-A1- 2012 219 952
- BOUTAL HERVé ET AL: "A multiplex lateral flow immunoassay for the rapid identification of NDM-, KPC-, IMP- and VIM-type and OXA-48-like carbapenemase-producing Enterobacteriaceae", JOURNAL OF ANTIMICROBIAL CHEMOTHERAPY, vol. 73, no. 4, 22 January 2018 (2018-01-22), pages 909-915, XP055841261, GB ISSN: 0305-7453, DOI: 10.1093/jac/dkx521
- LEE JI EUN ET AL: "Rapid detection of carbapenemase-producing Enterobacteriaceae (CPE) using a simple DNA extraction method and Loop-mediated isothermal amplification (LAMP)", JOURNAL OF MICROBIOLOGICAL METHODS, ELSEVIER, AMSTERDAM, NL, vol. 167, 5 November 2019 (2019-11-05), XP085921881, ISSN: 0167-7012, DOI: 10.1016/J.MIMET.2019.105746 [retrieved on 2019-11-05]
- LIMBAGO BRANDI M. ET AL: "IMP-Producing Carbapenem-Resistant Klebsiella pneumoniae in the United States", JOURNAL OF CLINICAL MICROBIOLOGY, vol. 49, no. 12, 1 December 2011 (2011-12-01), pages 4239-4245, XP093007396, US ISSN: 0095-1137, DOI: 10.1128/JCM.05297-11 Retrieved from the Internet: URL:https://www.ncbi.nlm.nih.gov/pmc/artic les/PMC3233008/pdf/zjm4239.pdf>
- ZARAKOLU PINAR ET AL: "Epidemiology of carbapenem-resistantKlebsiella pneumoniaecolonization: a surveillance study at a Turkish university hospital from 2009 to 2013", DIAGNOSTIC MICROBIOLOGY AND INFECTIOUS DISEASE, ELSEVIER, AMSTERDAM, NL, vol. 85, no. 4, 21 May 2016 (2016-05-21), pages 466-470, XP029632375, ISSN: 0732-8893, DOI: 10.1016/J.DIAGMICROBIO.2016.05.012
- NORIHISA YAMAMOTO ET AL: "Development of selective medium for IMP-type carbapenemase-producing in stool specimens", BMC INFECTIOUS DISEASES, BIOMED CENTRAL LTD, LONDON, UK, vol. 17, no. 1, 24 March 2017 (2017-03-24) , pages 1-5, XP021243517, DOI: 10.1186/S12879-017-2312-1
- DUBENDRIS HEATHER ET AL: "Imipenemase-producing carbapenem-resistant Enterobacteriaceae transmission in a long-term-care facility during a community-wide multidrug resistant organism outbreak-North Carolina, 2017", AJIC: AMERICAN JOURNAL OF INFECTION CONTROL, ELSEVIER, AMSTERDAM, NL, vol. 48, no. 3, 19 July 2019 (2019-07-19), pages 320-323, XP086056533, ISSN: 0196-6553, DOI: 10.1016/J.AJIC.2019.05.022 [retrieved on 2019-07-19]
- LOWE CHRISTOPHER F. ET AL: "The Brief Case: IMP, the Uncommonly Common Carbapenemase", JOURNAL OF CLINICAL MICROBIOLOGY, vol. 58, no. 4, 25 March 2020 (2020-03-25) , XP093007401, US ISSN: 0095-1137, DOI: 10.1128/JCM.01094-19 Retrieved from the Internet: URL:https://journals.asm.org/doi/pdf/10.11 28/JCM.01094-19>

## Description

### TECHNICAL FIELD

The present disclosure belongs to the technical field of antibody preparation, and in particular to an IMP-type carbapenemase-resistant hybridoma cell line, and a monoclonal antibody (mAb) and use thereof.

### BACKGROUND

Carbapenems are one of the most effective drugs for controlling clinical pathogen infections. Carbapenemase-producing organisms (CPOs) and carbapenem-resistant *Enterobacteriaceae* (CRE) have become a global public health issue due to their broad-spectrum drug resistance, and related patients have very limited treatment options. *Enterobacteriaceae* bacteria are important pathogens causing nosocomial infection and community-acquired infection. In recent years, due to the wide application of cephalosporins, a proportion of strains producing extended spectrum beta-lactamases (ESBLs) and AmpC enzymes in *Enterobacteriaceae* has been on the rise significantly, and carbapenem antibacterial drugs are the drugs of choice for treating infections of such strains. However, with the large-scale clinical use of imipenem and meropenem, the number of CRE is also increasing year by year. Clinical studies have shown that CRE strains are resistant to a variety of antibacterial drugs, and thus the choice of clinical treatment drugs is obviously limited. In addition, infected patients are often accompanied by concurrent diseases, which brings great difficulties to clinical infection control and treatment.

IMP-type enzymes are a very common type of acquired metallo-β-lactamases (MBLs), and belong to the subclass 3a of the class Bush. The IMP-type enzymes can hydrolyze almost all β-lactam antibacterial drugs, and the hydrolysis activity thereof is not inhibited by β-lactamase inhibitors such as clavulanic acid, sulbactam, and tazobactam. However, carbapenems can be inhibited by metal chelates such as ethylenediaminetetraacetic acid (EDTA) and sulfhydryl compounds, and in the absence of other coexisting resistance mechanisms, IMP-type carbapenemase-producing strains are still sensitive to monocyclic antibacterial drugs (aztreonam (ATM) and the like).

There are numerous methods for laboratory detection of carbapenemases. The methods mainly include modified Hodge test, Carba NP test, modified carbapenem inactivation method (mCIM), enzyme inhibitor enhancement test, immunogold labeling test, molecular biotechnology, and the like. Immunodiagnostic reagents are the fastest-growing segment of *in vitro* diagnostic reagents, which achieve qualitative or quantitative detection through specific binding between an antigen and an antibody.

Boutal Herve et. al, discloses a multiplex lateral flow immunoassay for the rapid identification of NDM-, KPC-, IMP- and VIM- type and OXA-48 like carbapenamase-producing Enterobacteriaceae. Its aim is to detect the presence of the 4 carbapenamase families with a sensitivity of 100% without any false negatives. However, it only discloses antibodies suitable for detecting IMP-1, IMP-8 and IMP-11. Furthermore, it does not disclose the limit of detection of the presence of the IMP-4 type carbapenamase and the minimum titer of antibody that can detected the presence of the antigen (BOUTAL HERVE; ET AL: "A multiplex lateral flow immunoassay for the rapid identification of NDM-, KPC-, IMP- and VIM-type and OXA-48-like carbapenemase- producing Enterobacteriaceae", JOURNAL OF ANTIMICROBIAL CHEMOTHERAPY, vol. 73, no. 4, 22 January 2018, pages 909-915)

### SUMMARY

The present disclosure provides an IMP-type carbapenemase-resistant hybridoma cell line, and a mAb and use thereof.

The present disclosure adopts the following technical solutions:
An anti-IMP-type carbapenemase antibody is provided, wherein the anti-IMP-type carbapenemase antibody is: an antibody 1AA4 including a light chain variable region and a heavy chain variable region, where complementary-determining regions (CDRs) of the light chain variable region include three sequences shown in SEQ ID NO: 1, SEQ ID NO: 2, and SEQ ID NO: 3; and CDRs of the heavy chain variable region include three sequences shown in SEQ ID NO: 4, SEQ ID NO: 5, and SEQ ID NO: 6;
   SEQ ID NO: 1
      RSSQSLLDSGGKTYLN (CDRL1)
   SEQ ID NO: 2
      YLVSKL (CDRL2)
   SEQ ID NO: 3
      FQYTHFPQCT (CDRL3)
   SEQ ID NO: 4
      AASGFTFSDYYMY (CDRH1)
   SEQ ID NO: 5
      VAIISDGGSYTYYPDSVKG (CDRH2)
   SEQ ID NO: 6
      VRGSFDV
   or,
an antibody AH9 including a light chain variable region and a heavy chain variable region, where CDRs of the light chain variable region include three sequences shown in SEQ ID NO: 11, SEQ ID NO: 12, and SEQ ID NO: 13; and CDRs of the heavy chain variable region include three sequences shown in SEQ ID NO: 14, SEQ ID NO: 15, and SEQ ID NO: 16.
   SEQ ID NO: 11
      KSSQSLLNSGHQKNYLA (CDRL1)
   SEQ ID NO: 12
      YGASTRK (CDRL2)
   SEQ ID NO: 13
      QNDHRYPLT (CDRL3)
   SEQ ID NO: 14
      KASGYTFTDYNMD (CDRH1)
   SEQ ID NO: 15
      IGDINPNYDSTRYNQKFKG (CDRH2)
   SEQ ID NO: 16
      ARDGRYAMDY (CDRH3)

Preferably, in the antibody 1AA4, the light chain variable region may have an amino acid sequence shown in SEQ ID NO: 7, and the heavy chain variable region may have an amino acid sequence shown in SEQ ID NO: 9;
SEQ ID NO: 7 amino acid sequence of light chain variable region 
DIVMTQSPLTLSVTLGQPASISCRSSQSLLDSGGKTYLNWFLQRPGQSPKRLIYLV
SKLDSGVPDRFTGSGSGTDFTLRISRVEAEDLGIYYCFQYTHFPQCTFGGGTTLEIKRA
SEQ ID NO: 9 amino acid sequence of heavy chain variable region 
VQLVVSGGGLVNPDGSLKLSCAASGFTFSDYYMYWVRQTPEKRLEWVAIISDGG
SYTYYPDSVKGRFTISRDNAKNNLYLQMSGLRSEDTAMYYCVRGSFDVWGAGTTVT
VSA
 or,
in the antibody AH9, the light chain variable region may have an amino acid sequence shown in SEQ ID NO: 17, and the heavy chain variable region may have an amino acid sequence shown in SEQ ID NO: 19.
SEQ ID NO: 17 amino acid sequence of light chain variable region 
DIVMTQSPSSLSVSAGEKVTLTCKSSQSLLNSGHQKNYLAWYQQKPGQPPKLLIY
GASTRKSGIPDRFTGSGSGTDFTLTISSVQAEDLAVYYCQNDHRYPLTFGAGTKLELK
RA
SEQ ID NO: 19 amino acid sequence of heavy chain variable region

VKLQQSGAELVKPGASVKISCKASGYTFTDYNMDWVKQSHGKSLEWIGDINPN
YDSTRYNQKFKGKATLTVDKSSSTAYMELRSLTSEDTAVYYCARDGRYAMDYWGQ
GTSVTVSS

Preferably, the antibodies may be produced by IMP-type carbapenemase-resistant hybridoma cell lines with accession numbers of CGMCC No. 20287 and CGMCC No. 20288, respectively.

A nucleic acid is provided, including a nucleotide sequence encoding the anti-IMP-type carbapenemase antibody described above.

Preferably, a nucleotide sequence of the nucleic acid for encoding the light chain variable region of the antibody 1AA4 may be as shown in SEQ ID NO: 8, and a nucleotide sequence of the nucleic acid for encoding the heavy chain variable region of the antibody 1AA4 may be as shown in SEQ ID NO: 10; and
SEQ ID NO: 8 base sequence for light chain variable region 
GACATTGTCATGACCCAGTCTCCACTCACTTTGTCGGTTACCCTTGGACAACC
AGCCTCCATCTCTTGCAGGTCAAGTCAGAGCCTCTTAGATAGTGGTGGAAAGACA
TATTTGAATTGGTTTTTACAGAGGCCAGGCCAGTCTCCAAAGCGCCTAATCTATCT
GGTGTCTAAACTGGACTCTGGAGTCCCTGACAGGTTCACTGGCAGTGGATCAGGG
ACAGATTTCACACTGAGAATCAGCAGAGTGGAGGCTGAGGATTTGGGAATTTATT
ATTGCTTCCAATATACACATTTTCCTCAGTGTACGTTCGGAGGGGGGACCACGCT
GGAAATCAAACGGGCT
SEQ ID NO: 10 base sequence for heavy chain variable region 
GTGCAGTTGGTGGTGTCTGGGGGAGGCTTAGTGAACCCTGACGGGTCCCTGA
AACTCTCCTGTGCAGCCTCTGGATTCACTTTCAGTGACTATTACATGTATTGGGTT
CGCCAGACTCCGGAAAAGAGGCTGGAGTGGGTCGCTATAATTAGTGATGGTGGTT
CTTACACCTACTATCCAGACAGTGTGAAGGGGCGATTCACCATCTCCAGAGACAA
TGCCAAGAATAATTTGTACCTGCAAATGAGCGGTCTGAGGTCTGAGGACACGGCC
ATGTATTATTGTGTAAGAGGGTCATTCGATGTCTGGGGCGCAGGGACCACGGTCA
CCGTCTCTGCAGC
 or,
a nucleotide sequence of the nucleic acid for encoding the light chain variable region of the antibody AH9 may be as shown in SEQ ID NO: 18, and a nucleotide sequence of the nucleic acid for encoding the heavy chain variable region of the antibody AH9 may be as shown in SEQ ID NO: 20.
SEQ ID NO: 18 base sequence for light chain variable region 
GACATTGTCATGACACAGTCTCCATCCTCCCTGAGTGTGTCAGCAGGAGAGA
AGGTCACTTTGACCTGCAAGTCCAGTCAGAGTCTGTTAAACAGTGGACATCAAAA
GAACTACTTGGCCTGGTACCAGCAGAAACCAGGGCAGCCTCCTAAACTGTTGATC
TACGGGGCATCCACTAGGAAATCTGGGATCCCTGATCGCTTCACAGGCAGTGGAT
CTGGAACCGATTTCACTCTTACCATCAGCAGTGTGCAGGCTGAAGACCTGGCAGT
TTATTACTGTCAGAATGATCATCGTTATCCTCTCACGTTCGGTGCTGGGACCAAGC
TGGAGCTGAAACGGGCTGA
SEQ ID NO: 20 base sequence for heavy chain variable region

GTGAAGCTTCAGCAGTCTGGAGCTGAGCTGGTGAAGCCTGGGGCTTCAGTGA
AGATATCCTGCAAGGCTTCTGGCTACACATTCACTGACTACAACATGGACTGGGT
GAAGCAGAGCCATGGAAAGAGCCTTGAGTGGATTGGAGATATTAATCCTAACTA
TGATAGTACTAGGTACAACCAGAAGTTCAAGGGAAAGGCCACATTGACTGTAGA
CAAGTCCTCCAGCACAGCCTACATGGAGCTCCGCAGCCTGACATCTGAGGACACT
GCAGTCTATTACTGTGCAAGAGACGGCCGCTATGCTATGGACTACTGGGGTCAAG
GAACCTCAGTCACCGTCTCTTCA

Use of the anti-IMP-type carbapenemase antibody described above in the detection of an IMP-type carbapenemase antigen is provided.

Preferably, the anti-IMP-type carbapenemase antibody may be used in an *in vitro* diagnostic kit or a microfluidic chip; and the *in vitro* diagnostic kit may be a colloidal gold immunoassay kit, a chemiluminescence kit, a radioimmunoassay kit, an enzyme-linked immunosorbent assay (ELISA) kit, or a fluoroimmunoassay kit.

Preferably, an immune colloidal gold test strip of a double-antibody sandwich method may be prepared, wherein the antibody 1AA4 is used as a coating antibody and the antibody AH9 is used as a gold-labeled antibody; or the antibody AH9 is used as a coating antibody and the antibody 1AA4 is used as a gold-labeled antibody.

The present disclosure has the following advantages and positive effects: an anti-IMP-type carbapenemase antibody is provided, which can be used to detect IMP-type carbapenemase, with a titer of more than 1 :640,000; an *in vitro* diagnostic kit or a microfluidic chip prepared therefrom can be used for early typing of drug-resistant strains, guidance of drug use, and assistance of clinical infection control and treatment.

### BRIEF DESCRIPTION OF THE DRAWINGS

FIG. 1 is an electrophoretogram of the IMP-type carbapenemase protein; and
FIG. 2 is an electrophoretogram of the anti-IMP-type carbapenemase antibody protein.

### DETAILED DESCRIPTION OF THE EMBODIMENTS

The embodiments of the present disclosure will be described below.

The present disclosure relates to an anti-IMP-type carbapenemase antibody produced using an IMP-type carbapenemase-resistant hybridoma cell line named 1AA4, which belongs to hybridoma cells. The present disclosure also relates to an anti-IMP-type carbapenemase antibody produced using an IMP-type carbapenemase-resistant hybridoma cell line named AH9, which belongs to hybridoma cells.

The IMP-type carbapenemase-resistant hybridoma cell line capable of stably secreting an anti-IMP-type carbapenemase antibody and a variable region sequence thereof provided by the present disclosure are obtained through mouse hybridoma mAb screening and reverse transcription-polymerase chain reaction (RT-PCR) to clone an Ig variable region gene, and the binding specificity of the antibody is identified by ELISA.

An antibody 1AA4 produced by the IMP-type carbapenemase-resistant hybridoma cell line includes a light chain variable region and a heavy chain variable region, where CDRs of the light chain variable region include three sequences shown in SEQ ID NO: 1, SEQ ID NO: 2, and SEQ ID NO: 3;
SEQ ID NO: 1
   RSSQSLLDSGGKTYLN (CDRL1)
SEQ ID NO: 2
   YLVSKL (CDRL2)
SEQ ID NO: 3
   FQYTHFPQCT (CDRL3)
   CDRs of the heavy chain variable region include three sequences shown in SEQ ID NO: 4, SEQ ID NO: 5, and SEQ ID NO: 6;
SEQ ID NO: 4
   AASGFTFSDYYMY (CDRH1)
SEQ ID NO: 5
   VAIISDGGSYTYYPDSVKG (CDRH2)
SEQ ID NO: 6
   VRGSFDV
   the light chain variable region may have an amino acid sequence shown in SEQ ID NO: 7,
SEQ ID NO: 7 
DIVMTQSPLTLSVTLGQPASISCRSSQSLLDSGGKTYLNWFLQRPGQSPKRLIYLV
SKLDSGVPDRFTGSGSGTDFTLRISRVEAEDLGIYYCFQYTHFPQCTFGGGTTLEIKRA
 the heavy chain variable region may have an amino acid sequence shown in SEQ ID NO: 9;
SEQ ID NO: 9 
VQLVVSGGGLVNPDGSLKLSCAASGFTFSDYYMYWVRQTPEKRLEWVAIISDGG
SYTYYPDSVKGRFTISRDNAKNNLYLQMSGLRSEDTAMYYCVRGSFDVWGAGTTVT
VSA
 a nucleotide sequence encoding the light chain variable region of the anti-IMP-type carbapenemase antibody may be as shown in SEQ ID NO: 8; and
SEQ ID NO: 8 
GACATTGTCATGACCCAGTCTCCACTCACTTTGTCGGTTACCCTTGGACAACC
AGCCTCCATCTCTTGCAGGTCAAGTCAGAGCCTCTTAGATAGTGGTGGAAAGACA
TATTTGAATTGGTTTTTACAGAGGCCAGGCCAGTCTCCAAAGCGCCTAATCTATCT
GGTGTCTAAACTGGACTCTGGAGTCCCTGACAGGTTCACTGGCAGTGGATCAGGG
ACAGATTTCACACTGAGAATCAGCAGAGTGGAGGCTGAGGATTTGGGAATTTATT
ATTGCTTCCAATATACACATTTTCCTCAGTGTACGTTCGGAGGGGGGACCACGCT
GGAAATCAAACGGGCT
 a nucleotide sequence encoding the heavy chain variable region of the anti-IMP-type carbapenemase antibody may be as shown in SEQ ID NO: 10.
SEQ ID NO: 10

GTGCAGTTGGTGGTGTCTGGGGGAGGCTTAGTGAACCCTGACGGGTCCCTGA
AACTCTCCTGTGCAGCCTCTGGATTCACTTTCAGTGACTATTACATGTATTGGGTT
CGCCAGACTCCGGAAAAGAGGCTGGAGTGGGTCGCTATAATTAGTGATGGTGGTT
CTTACACCTACTATCCAGACAGTGTGAAGGGGCGATTCACCATCTCCAGAGACAA
TGCCAAGAATAATTTGTACCTGCAAATGAGCGGTCTGAGGTCTGAGGACACGGCC

ATGTATTATTGTGTAAGAGGGTCATTCGATGTCTGGGGCGCAGGGACCACGGTCA
CCGTCTCTGCAGC

An anti-IMP-type carbapenemase antibody produced by the IMP-type carbapenemase-resistant hybridoma cell line AH9 includes a light chain variable region and a heavy chain variable region, where CDRs of the light chain variable region include three sequences shown in SEQ ID NO: 11, SEQ ID NO: 12, and SEQ ID NO: 13;
SEQ ID NO: 11
   KSSQSLLNSGHQKNYLA (CDRL1)
SEQ ID NO: 12
   YGASTRK (CDRL2)
SEQ ID NO: 13
   QNDHRYPLT (CDRL3)
   CDRs of the heavy chain variable region include three sequences shown in SEQ ID NO: 14, SEQ ID NO: 15, and SEQ ID NO: 16;
SEQ ID NO: 14
   KASGYTFTDYNMD (CDRH1)
SEQ ID NO: 15
   IGDINPNYDSTRYNQKFKG (CDRH2)
SEQ ID NO: 16
   ARDGRYAMDY (CDRH3)
   the light chain variable region may have an amino acid sequence shown in SEQ ID NO: 17;
SEQ ID NO: 17 amino acid sequence of light chain variable region 
DIVMTQSPSSLSVSAGEKVTLTCKSSQSLLNSGHQKNYLAWYQQKPGQPPKLLIY
GASTRKSGIPDRFTGSGSGTDFTLTISSVQAEDLAVYYCQNDHRYPLTFGAGTKLELK
RA
 the heavy chain variable region may have an amino acid sequence shown in SEQ ID NO: 19;
SEQ ID NO: 19 amino acid sequence of heavy chain variable region 
VKLQQSGAELVKPGASVKISCKASGYTFTDYNMDWVKQSHGKSLEWIGDINPN
YDSTRYNQKFKGKATLTVDKSSSTAYMELRSLTSEDTAVYYCARDGRYAMDYWGQ
GTSVTVSS
 a nucleotide sequence encoding the light chain variable region of the anti-IMP-type carbapenemase antibody may be as shown in SEQ ID NO: 18; and
SEQ ID NO: 18 
GACATTGTCATGACACAGTCTCCATCCTCCCTGAGTGTGTCAGCAGGAGAGA
AGGTCACTTTGACCTGCAAGTCCAGTCAGAGTCTGTTAAACAGTGGACATCAAAA
GAACTACTTGGCCTGGTACCAGCAGAAACCAGGGCAGCCTCCTAAACTGTTGATC
TACGGGGCATCCACTAGGAAATCTGGGATCCCTGATCGCTTCACAGGCAGTGGAT
CTGGAACCGATTTCACTCTTACCATCAGCAGTGTGCAGGCTGAAGACCTGGCAGT
TTATTACTGTCAGAATGATCATCGTTATCCTCTCACGTTCGGTGCTGGGACCAAGC
TGGAGCTGAAACGGGCTGA
 a nucleotide sequence encoding the heavy chain variable region of the anti-IMP-type carbapenemase antibody may be as shown in SEQ ID NO: 20.
SEQ ID NO: 20

GTGAAGCTTCAGCAGTCTGGAGCTGAGCTGGTGAAGCCTGGGGCTTCAGTGA
AGATATCCTGCAAGGCTTCTGGCTACACATTCACTGACTACAACATGGACTGGGT
GAAGCAGAGCCATGGAAAGAGCCTTGAGTGGATTGGAGATATTAATCCTAACTA
TGATAGTACTAGGTACAACCAGAAGTTCAAGGGAAAGGCCACATTGACTGTAGA
CAAGTCCTCCAGCACAGCCTACATGGAGCTCCGCAGCCTGACATCTGAGGACACT
GCAGTCTATTACTGTGCAAGAGACGGCCGCTATGCTATGGACTACTGGGGTCAAG
GAACCTCAGTCACCGTCTCTTCA

The anti-IMP-type carbapenemase antibodies produced by the hybridoma cell line 1AA4 and the hybridoma cell line AH9 can be used to detect an IMP-type carbapenemase antigen. The anti-IMP-type carbapenemase mAb shows prominent performance in all aspects, and thus is suitable as an immunodiagnostic reagent for the preparation of an *in vitro* diagnostic kit, and the *in vitro* diagnostic kit may be a colloidal gold immunoassay kit, a chemiluminescence kit, a radioimmunoassay kit, an ELISA kit, or a fluoroimmunoassay kit. Alternatively, the anti-IMP-type carbapenemase antibody can be made into a microfluidic chip for detecting an IMP-type carbapenemase antigen.

To develop an IMP-type carbapenemase diagnostic reagent of the colloidal gold method, an IMP-type carbapenemase antigen first needs to be extracted to obtain a high-purity antigen, then the antigen is used to stimulate an excellent immune response in mice, and then the hybridoma technique is used to screen an antibody with high affinity and specificity for the development of related *in vitro* diagnostic reagents. The two antibodies involved in this solution are especially suitable for preparing an immune colloidal gold test strip of a double-antibody sandwich method, wherein the antibody 1AA4 is used as a coating antibody and the antibody AH9 is used as a gold-labeled antibody. The prepared immune colloidal gold test strip of a double-antibody sandwich method is more sensitive. Moreover, the antibody 1AA4 can be used as a gold-labeled antibody, and the antibody AH9 can be used as a coating antibody. The present disclosure is further described below through specific examples. Experimental methods for which operation steps are not specified are all conducted in accordance with the corresponding product instructions. The instruments, reagents, and consumables used in the examples can be purchased from commercial companies unless otherwise specified.

### Example 1: Preparation of an anti-IMP-type carbapenemase antibody

### 1.1 Antigen preparation

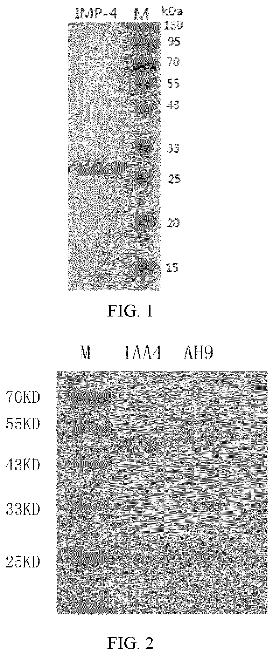

An IMP-4-type gene sequence was downloaded from National Center for Biotechnology Information (NCBI), subjected to gene synthesis, and ligated to a pet-28a vector, and then the vector plasmid pet-28a-IMP-4 carrying the target gene was transformed into an expression host *Rosseta* (DE3). The host was cultivated until an OD value of a bacterial solution was of 0.4 to 0.6, then isopropylthio-β-galactoside (IPTG) was added at a final concentration of 1 mM for induction, and the host was further cultivated at 37°C for 3 hours. Resulting bacterial cells were collected by centrifugation, then resuspended by adding 30 mL of phosphate buffered saline (PBS) to every about 1 g of the bacterial cells, and then subjected to ultrasonication at a power of 400 W for about 30 min (ultrasonic time: 3 s, interval: 5 s); after a bacterial solution was non-viscous and clear, the bacterial solution was centrifuged to remove bacterial debris, and a resulting supernatant was filtered through a 0.45 µm filter membrane and loaded on a nickel column packed and equilibrated with an equilibration buffer in advance. After the loading was completed, gradient elution was conducted with imidazole eluents and washing liquids at concentrations of 20 mM, 50 mM, 100 mM, 150 mM, 250 mM, and 500 mM. When the imidazole concentration was 250 mM and 500 mM, a target protein with high purity could be obtained, and a molecular weight thereof was consistent with the expected 29.6 kD. A sodium dodecyl sulphate-polyacrylamide gel electrophoresis (SDS-PAGE) result was shown in FIG. 1. The protein was quantified by bicinchoninic acid (BCA) assay and then dispensed.

### 1.2 Mouse immunization

6-week-old female Balb/c mice were immunized with the purified IMP-type carbapenemase to prepare an antibody, with a protein content of 0.1 mg/ml. The mice were divided into 2 groups according to the immunization dosage, with 5 mice in each group; according to a protein content, a first group was immunized at an immunization dosage of 25 µg/mouse, and a second group was immunized at an immunization dosage of 50 µg/mouse. For an initial immunization, an appropriate amount of the IMP-type carbapenemase was taken and diluted with distilled water to 300 µL, then 300 µL of complete Freund's adjuvant (CFA) was added for thorough emulsification, and a resulting emulsion was subcutaneously injected into the mice (multi-point injection); two weeks later, the mice were subjected to a secondary immunization by intraperitoneal injection at the same dosage; two weeks later, the mice were subjected to an additional immunization by intraperitoneal injection; 7 days later, blood was collected from the tail of mice, and a serum titer of the mice was determined by ELISA. Specific steps were as follows: An ELISA plate was coated overnight at 4°C with the IMP-type carbapenemase of 0.2 µg/ml at 100 µL/well, spin-dried, and washed 3 times with phosphate-buffered saline with Tween 20 (PBST). 5% skimmed milk powder was added at 200 µL/well to block at 37°C for 2 hours. Blood was collected from the tail of the mice and then centrifuged at 3,000 rpm to obtain serum, and the serum was serially diluted from 1: 1,000 to 1:512,000 with PBS for later use. The plate was spin-dried and then washed 3 times with PBST, a primary antibody diluted with PBS at 1:1,000 was added at 100 µL/well, and the plate was incubated at 37°C for 1 hour. The plate was spin-dried and then washed 3 times with PBST, a goat anti-mouse secondary antibody diluted with PBS at 1:6,000 was added at 100 µL/well, and the plate was incubated at 37°C for 45 min. The plate was spin-dried and then washed 5 times with PBST, 3,3',5,5'-tetramethylbenzidine (TMB) was added at 100 µL/well, and the plate was incubated at 37°C to allow a chromogenic reaction for 10 min; the chromogenic reaction was stopped, and a value was read.

### 1.3 Cell fusion

Three days before fusion, mice were subjected to a booster immunization by intraperitoneal injection at the same dosage as the previous immunization, without an adjuvant. Feeder cells were prepared one day before fusion. A Balb/c mouse 6-8 weeks old was selected, and eyeballs of the mouse were removed to collect blood; then the mouse was sacrificed by cervical dislocation, disinfected in 75% alcohol for 5 min, and fixed on a tray; the abdominal skin was aseptically cut in a clean bench. 10 ml of an HAT selection medium was injected into the abdominal cavity of the mouse with a sterile syringe, the abdomen was slightly rubbed with an alcohol cotton ball, and the medium was drawn out. The medium was added to 40 ml of an HAT medium, and a resulting mixture was added to 4 96-well cell culture plates at 100 µL/well, and then cultivated in a 37°C and 5% CO₂ incubator. One week before fusion, myeloma cells (Sp2/0 cells) were recovered, cultivated in a PRMI-1640 medium with 10% fetal bovine serum (FBS), and subcultivated in a 37°C and 5% CO₂ incubator. Cells in a logarithmic growth phase were collected into a centrifuge tube, subjected to cell counting, and diluted to 10⁷ cells/ml for later use. The Balb/c mice that had been subjected to the booster immunization 3 days prior were taken, eyeballs were removed to collect blood and prepare positive serum, and the mice were sacrificed by cervical dislocation and disinfected with 75% alcohol for 5 min; then a spleen was aseptically collected in a clean bench and washed several times in a sterile dish, and a connective tissue was stripped. The spleen was placed on a microporous copper mesh, a fresh RPMI-1640 medium was added, and the medium was first drawn with a syringe and injected from an end of the spleen to blow off spleen cells; after the process was repeated several times, an inner plug of the syringe was used to gently grind the remaining spleen until there was no obvious red tissue mass. A spleen cell suspension in a dish was gently pipetted up and down, then transferred to a 50 ml centrifuge tube, and centrifuged at 1,000 r/min for 5 min to collect spleen cells, and the spleen cells were counted for later use. The immunized mouse spleen cells and Sp2/0 cells were mixed at a cell number ratio of 10:1, added to a 50 ml centrifuge tube, and centrifuged at 1,000 r/min for 5 min, a resulting supernatant was discarded, and the two cells were thoroughly mixed by rubbing gently in the palm of hands. The centrifuge tube was placed in a 100 mL blue-cap bottle filled with 37°C hot water, and then 1 ml of preheated dimethyl sulfoxide (DMSO)/polyethylene glycol (PEG) was added dropwise to the fusion tube within 1 min, during which the DMSO/PEG was added slowly first and then quickly, and the centrifuge tube was gently swirled. Then an antibiotic-free and serum-free RPMI-1640 medium was immediately added to stop the reaction, during which 1 ml of the medium was added in the first minute, 2 ml of the medium was added in the second minute, 3 ml of the medium was added in the third minute, and 4 ml of the medium was added in the fourth minute. A resulting mixture was incubated in a 37°C water bath for 5 min and then centrifuged at 800 r/min for 5 min, and a resulting supernatant was discarded; a resulting precipitate was suspended with HAT, then thoroughly mixed with 40 ml of an HAT selection medium including 20% calf serum preheated at 37°C, and added to a 96-well cell plate with feeder cells at 100 µL/well, and the cell plate was incubated in a 37°C and 5% CO₂ incubator. 7 days later, half of the medium in the cell plate was replaced with fresh HAT medium, and 10 days later, the medium was completely replaced with HT medium. Positive cells in the 96-well plate were subcloned by limiting dilution analysis (LDA): feeder cells were prepared first according to the above method, and hybridoma cells to be cloned were taken and counted, then diluted to 5 to 8 cells/ml with HT medium, added at 100 µL/well to a 96-well cell plate with the feeder cells (the cloning of each hybridoma cell line required a 96-well cell plate), and cultivated in a 37°C and 5% CO₂ incubator; the number of clones in each cell well was counted and marked about 5 days later, the medium was replaced with fresh medium on day 7, and a test was conducted when cells covered 1/3 to 1/2 of the entire bottom surface of each well; after 2 to 3 times of cloning, all cell wells of the 96-well plate were positive, at which point the cells could be expanded, verified, and cryopreserved. Hybridoma cells verified as positive were expanded and cryopreserved. A specific process was as follows: vigorously-growing hybridoma cells in prominent conditions were gently blown off from a flask with antibiotic-free and serum-free DMEM and then centrifuged at 1,000 r/min for 5 min, and a resulting supernatant was discarded; a cryopreservation solution (with 40% RPMI-1640 medium, 50% FBS, and 10% DMSO) was added to make cells uniformly dispersed, and a resulting cell suspension was then dispensed into cell cryopreservation tubes; then the cryopreservation tubes were placed in a cryopreservation box, the cryopreservation box was placed in a -70°C freezer, and one day later, the cryopreservation tubes were transferred to liquid nitrogen and related records were made.

### 1.4 Ascitic fluid preparation

10 to 12-week-old female Balb/c mice were injected intraperitoneally with sterile liquid paraffin at 0.5 mL/mouse, and 7 days later, the mice were injected intraperitoneally with hybridoma cells in a logarithmic growth phase at 5 × 10⁶ cells/mouse. The mice were observed every day. About 7 to 10 days later, abdomens of the mice obviously bulged, at which point the lower abdominal skin was disinfected with a 75% alcohol cotton ball and then the abdominal cavity was punctured with a 16-gauge needle to collect an ascitic fluid. After an ascitic fluid was regenerated and accumulated, the ascitic fluid was collected once again. The collected ascitic fluid was centrifuged at 3,000 r/min for 10 min, and a resulting clear middle layer was collected, filtered with filter paper, dispensed, and stored at -70°C.

### 1.5 Antibody purification

The ascitic fluid was purified with a Protein-G column. Specific steps were as follows: 2 ml (n) of the ascitic fluid was taken and centrifuged at 10,000 g, and a resulting clear layer was collected and thoroughly mixed with 2 ml of a washing buffer (1:1) to obtain a sample; 20% ethanol was passed through the column, then the column was equilibrated with 8 mL of a washing liquid, and the sample was passed through the column at a flow rate of 8 S/drop; the sample was repeatedly loaded 3 times, and then the column was washed with 15 mL of a washing buffer at a flow rate of 8 S/drop; after the washing was completed, elution was conducted with 10 mL of an elution buffer, and after the elution was completed, a pH was adjusted to 7.4 with 1 M Tris PH = 9; then concentration was conducted with a concentration column, and a resulting concentrate was subjected to dialysis overnight at 4°C with PBS in a 50 kD dialysis bag to obtain an antibody 1AA4 and an antibody AH9.

### Example 2: Identification of the anti-IMP-type carbapenemase antibody

### 2.1 Antibody subtype identification

According to the instructions of the SIGMA kit, the mAb subtype identification was conducted by capture-ELISA. Specific steps were as follows:
an mAb subtype identification reagent was diluted at 1:1,000, added to an ELISA plate at 100 µL/well, and incubated at 37°C for 1 h; the plate was washed three times with PBST and pat-dried; the antibody was diluted at 1:1,000 and added at 100 µL/well, and then the plate was incubated at 37°C for 1 h; the plate was washed three times with PBST and pat-dried; an HRP enzyme-labeled goat anti-mouse IgG secondary antibody was diluted at 1:6,000 and added at 100 µL/well, and then the plate was incubated at room temperature for 30 min; a chromogenic reaction was conducted for 10 min to 20 min. A subtype reagent added in a well with an OD450 reading significantly higher than that of other wells indicated the subtype of the mAb. The antibody subtype of the antibodies 1AA4 and AH9 was identified as IgG1.

### 2.2 Antibody titer determination

The antibody titer was determined after purification was conducted by indirect ELISA. Specific steps were as follows: IMP-4-type carbapenemase was diluted to 0.2 µg/mL and added to an ELISA plate at 100 µL/well to coat overnight at 4°C, where an uncoated control was set; the plate was spin-dried and then washed 3 times with PBST; 5% skimmed milk powder was added at 200 µL/well to block at 37°C for 2 h; the plate was spin-dried and then washed 3 times with PBST; the antibody (with a concentration of 1 mg/ml) was serially diluted from 1:1,000 to obtain a total of 12 gradient concentrations, resulting antibody dilutions were each added to the plate at 100 µL/well, and the plate was incubated at 37°C for 1 h, where an uncoated control was set; the plate was spin-dried and then washed 3 times with PBST, a goat anti-mouse secondary antibody diluted with PBS at 1:6,000 was added at 100 µL/well, and the plate was incubated at 37°C for 45 min; the plate was spin-dried and then washed 5 times with PBST, TMB was added at 100 µL/well, and the plate was incubated at 37°C to allow a chromogenic reaction for 10 min; the chromogenic reaction was stopped, and a value was read. After the purification, when the two antibodies were diluted to 1 mg/ml, the two both could show a titer of more than 1 :640,000.

### 2.3 Identification of antibody purity and molecular weight

SDS-PAGE was used to identify the molecular weight and purity of the antibody. Gels were prepared, where a separation gel was 12% and a stacking gel was 5%; 20 µL of a sample and 20 µL of a buffer were thoroughly mixed and boiled for 3 min to obtain a sample solution; 20 µL of the sample solution was loaded in each well, and a prestained protein Marker control was set; electrophoresis was conducted at 80 V for 30 min and at 120 V for 2 h; after the electrophoresis was completed, a Coomassie brilliant blue (CBB) solution was added for staining; destaining was conducted by boiling in deionized water 3 times, with 5 min each time; a purified mAb was identified by SDS-PAGE, and resulting bands were clear, without impurity bands. As shown in FIG. 2, there were clear bands at 50 KDa and 25 KDa for the two antibodies.

### Example 3: Gene verification of the anti-IMP-type carbapenemase antibody

An Ig variable region gene was cloned by RT-PCR. Total RNA was extracted to synthesize single-stranded cDNA. Total RNA was extracted from the hybridoma cell lines 1AA4 and AH9 by the Trizol method (purchased from Invitrogen), and was reverse-transcribed into a cDNA library with M-MLV reverse transcriptase (purchased from Invitrogen).
Upstream primer for a heavy chain framework region
   P1: 5'SAGGTGMAGCTKCASSARTCWGG3', as shown in SEQ ID NO: 21
Downstream primer for a heavy chain variable region
   P2: 5'TGGGGSTGTYGTTTTGGCTGMRGAGACRGTGA3', as shown in SEQ ID NO: 22
Upstream primer for a light chain leader peptide
   P3: 5'ATGGATTTTCAAGTGCAGATTTTCAG3', as shown in SEQ ID NO: 23
Downstream primer for a light chain variable region
   P4: 5'GGATACAGTTGGTGCAGCATCAGCCCGTTT3', as shown in SEQ ID NO: 24

A PCR system (50 µL) was prepared as follows:
cDNA: 2 µL; upstream primer (10 µM): 2 µL; downstream primer (10 µM): 2 µL; dNTP mixture: 2 µL; pfu DNA polymerase (5 U/µL): 1 µL; 10 X pfu Buffer II: 5 µL; and ddH₂O: making up to 50 µL.

PCR conditions were as follows: pre-denaturation at 95°C for 5 min; repeating the following cycle 35 times: 95°C for 30 s, 58°C for 30 s, and 72°C for 1 min; and finally, extension at 72°C for 10 min.

VL and VH fragments were separated and recovered by agarose gel electrophoresis. The recovered VL and VH fragments were ligated with a pMD19-T (simple) vector (Takara), and a ligation system was as follows:
VL PCR product/VH PCR product: each 70 ng,
pMD19-T (simple) vector: 1 µL,
Solution I ligation reaction solution: 5 µL, and
ddH₂O: making up to 10 µL.
The ligation was conducted at 4°C overnight.

A ligation product was transformed into *Escherichia coli* (*E. coli*) DH5α competent cells, the competent cells were cultivated overnight at 37°C, single colonies were picked and cultivated at 37°C under shaking for 2 h, and then a resulting bacterial solution was subjected to PCR identification. cDNA of a corresponding antibody was used as a positive control. A reaction system (25 µL) was prepared as follows:
bacterial solution: 1 µL,
upstream primer (10 µM): 1 µL,
downstream primer (10 µM): 1 µL,
dNTP Mixture (each 2.5 Mm): 2 µL,
Taq DNA polymerase (5 U/µL): 0.5 µL,
10 × Taq Buffer (Mg2 + plus): 2.5 µL; and
water: making up to 25 µL.

Reaction conditions were the same as above.

PCR-positive clones were selected for expansion, and a plasmid was extracted from the positive clones with a plasmid extraction kit (Takara) and sequenced. At least 5 clone samples were sequenced for each chain of each antibody until at least three samples had the same sequencing result. The heavy chain and light chain variable region sequences of the antibodies 1AA4 and AH9 were successfully cloned, which were consistent with the characteristics of typical antibody variable region sequences according to alignment.

### Example 4: Preparation of an IMP-type carbapenemase detection kit

An IMP-type carbapenemase test card was prepared by a colloidal gold method, and an immune colloidal gold test strip of a double-antibody sandwich method was prepared as follows:
step 1: a 0.1 M K₂CO₃ solution was added to a colloidal gold solution under stirring, a pH was adjusted, and the anti-IMP-type carbapenemase mAb AH9 was added; a resulting mixture was stirred, a 10% bovine serum albumin (BSA) solution and 2% PEG 20000 were added, and a resulting mixture was stirred and centrifuged at a low speed to obtain a supernatant; then the supernatant was centrifuged at a high speed to obtain a precipitate, and the precipitate was resuspended with colloidal gold to form a gold-labeled antibody;
step 2: the gold-labeled antibody was sprayed on a glass fiber membrane, and then the glass fiber membrane was oven-dried to form a gold-labeled pad;
step 3: a 1% thimerosal sodium solution was thoroughly mixed with the anti-IMP-type carbapenemase mAb 1AA4 to form a test line coating solution; then PBS and a 1% thimerosal sodium solution were added to the goat anti-mouse IgG, and a resulting mixture was thoroughly mixed to form a control line coating solution; the control line coating solution and the test line coating solution were streaked on a nitrocellulose (NC) membrane, and the NC membrane was oven-dried to obtain a coated membrane; and
step 4: the coated membrane was attached to a backing card, the gold-labeled pad and absorbent paper were lapped on the coated membrane, and a resulting product was laminated and cut to obtain the IMP-type carbapenemase test card of the colloidal gold method.

The examples of the present disclosure are described above in detail, which are merely preferred examples of the present disclosure and cannot be construed as limiting the scope of implementation of the present disclosure.

## Claims

1. An anti-IMP-type carbapenemase antibody, wherein the anti-IMP-type carbapenemase antibody is: an antibody 1AA4 comprising a light chain variable region and a heavy chain variable region, wherein complementary-determining regions (CDRs) of the light chain variable region comprise three sequences shown in SEQ ID NO: 1, SEQ ID NO: 2, and SEQ ID NO: 3; and CDRs of the heavy chain variable region comprise three sequences shown in SEQ ID NO: 4, SEQ ID NO: 5, and SEQ ID NO: 6;
or,
an antibody AH9 comprising a light chain variable region and a heavy chain variable region, wherein CDRs of the light chain variable region comprise three sequences shown in SEQ ID NO: 11, SEQ ID NO: 12, and SEQ ID NO: 13; and CDRs of the heavy chain variable region comprise three sequences shown in SEQ ID NO: 14, SEQ ID NO: 15, and SEQ ID NO: 16.

2. The anti-IMP-type carbapenemase antibody according to claim 1, wherein in the antibody 1AA4, the light chain variable region has an amino acid sequence shown in SEQ ID NO: 7, and the heavy chain variable region has an amino acid sequence shown in SEQ ID NO: 9;
or,
in the antibody AH9, the light chain variable region has an amino acid sequence shown in SEQ ID NO: 17, and the heavy chain variable region has an amino acid sequence shown in SEQ ID NO: 19.

3. A nucleic acid, comprising a nucleotide sequence encoding the anti-IMP-type carbapenemase antibody according to claim 1 or 2.

4. The nucleic acid according to claim 3,
wherein a nucleotide sequence of the nucleic acid for encoding the light chain variable region of the antibody 1AA4 is shown in SEQ ID NO: 8, and a nucleotide sequence of the nucleic acid for encoding the heavy chain variable region of the antibody 1AA4 is shown in SEQ ID NO: 10;
or,
a nucleotide sequence of the nucleic acid for encoding the light chain variable region of the antibody AH9 is shown in SEQ ID NO: 18, and a nucleotide sequence of the nucleic acid for encoding the heavy chain variable region of the antibody AH9 is shown in SEQ ID NO: 20.

5. Use of the anti-IMP-type carbapenemase antibody according to claim 1 or 2 in the in vitro detection of an IMP-type carbapenemase antigen.

6. The use of the anti-IMP-type carbapenemase antibody in the detection of an IMP-type carbapenemase antigen according to claim 5. wherein the anti-IMP-type carbapenemase antibody is used in an *in vitro* diagnostic kit or a microfluidic chip; and the *in vitro* diagnostic kit is a colloidal gold immunoassay kit, a chemiluminescence kit, a radioimmunoassay kit, an enzyme-linked immunosorbent assay (ELISA) kit, or a fluoroimmunoassay kit.

7. The use of the anti-IMP-type carbapenemase antibody in the detection of an IMP-type carbapenemase antigen according to claim 5.
wherein an immune colloidal gold test strip of a double-antibody sandwich method is prepared, wherein the antibody 1AA4 is used as a coating antibody and the antibody AH9 is used as a gold-labeled antibody; or
the antibody AH9 is used as a coating antibody and the antibody 1AA4 is used as a gold-labeled antibody.

## Patentansprüche

1. Carbapenemase-Antikörper vom Anti-IMP-Typ, wobei der Carbapenemase-Antikörper vom Anti-IMP-Typ ein Antikörper 1AA4 ist, umfassend eine variable Region der leichten Kette und eine variable Region der schweren Kette, wobei die komplementärbestimmenden Regionen (CDRs) der variablen Region der leichten Kette drei Sequenzen umfassen, die gezeigt sind in SEQ ID NO: 1, SEQ ID NO: 2, und SEQ ID NO: 3; und die CDRs der variablen Region der schweren Kette umfassen drei Sequenzen, die gezeigt ist in SEQ ID NO: 4, SEQ ID NO: 5, und SEQ ID NO: 6;
oder
einen Antikörper AH9, der eine variable Region der leichten Kette und eine variable Region der schweren Kette umfasst, wobei die CDRs der variablen Region der leichten Kette drei Sequenzen umfassen, welche gezeigt sind in SEQ ID NO: 11, SEQ ID NO: 12, und SEQ ID NO: 13; und die CDRs der variablen Region der schweren Kette umfassen drei Sequenzen, die gezeigt ist in SEQ ID NO: 14, SEQ ID NO: 15, und SEQ ID NO: 16.

2. Anti-IMP-Typ-Carbapenemase-Antikörper nach Anspruch 1, wobei in dem Antikörper 1AA4 die variable Region der leichten Kette eine Aminosäuresequenz aufweist, die gezeigt ist in SEQ ID NO: 7, und die variable Region der schweren Kette eine Aminosäuresequenz aufweist, die gezeigt ist in SEQ ID NO: 9;
oder
im Antikörper AH9 weist die variable Region der leichten Kette eine Aminosäuresequenz auf, die gezeigt ist, in SEQ ID NO: 17, und die variable Region der schweren Kette eine Aminosäuresequenz aufweist, die gezeigt ist in SEQ ID NO: 19.

3. Eine Nukleinsäure, umfassend eine Nukleotidsequenz, die den Anti-IMP-Typ-Carbapenemase-Antikörper nach Anspruch 1 oder 2 kodiert.

4. Nukleinsäure nach Anspruch 3, wobei eine Nukleotidsequenz der Nukleinsäure zum Kodieren der variablen Region der leichten Kette des Antikörpers 1AA4, die gezeigt ist in SEQ ID NO: 8, und eine Nukleotidsequenz der Nukleinsäure zum Kodieren der variablen Region der schweren Kette des Antikörpers 1AA4, die gezeigt ist in SEQ ID NO: 10;
oder
eine Nukleotidsequenz der Nukleinsäure zum Kodieren der variablen Region der leichten Kette des Antikörpers AH9, die gezeigt ist in SEQ ID NO: 18, und eine Nukleotidsequenz der Nukleinsäure, die für die variable Region der schweren Kette des Antikörpers AH9 kodiert, die gezeigt ist in SEQ ID NO: 20.

5. Anwendung des Anti-IMP-Typ-Carbapenemase-Antikörpers nach Anspruch 1 oder 2 bei dem In-vitro-Nachweis eines IMP-Typ-Carbapenemase-Antigens.

6. Anwendung des Anti-IMP-Typ-Carbapenemase-Antikörpers zum Nachweis eines IMP-Typ-Carbapenemase-Antigens nach Anspruch 5,
wobei der Anti-IMP-Typ-Carbapenemase-Antikörper in einem In-vitro-Diagnostik-Kit oder einem mikrofluidischen Chip verwendet wird; und das In-vitro-Diagnostik-Kit ein Kolloidalgold-Immunoassay-Kit, ein Chemolumineszenz-Kit, ein Radioimmunoassay-Kit, ein Enzyme-Linked-Immunosorbent-Assay (ELISA)-Kit oder ein Fluorimmunoassay-Kit ist.

7. Anwendung des Anti-IMP-Typ-Carbapenemase-Antikörpers zum Nachweis eines IMP-Typ-Carbapenemase-Antigens nach Anspruch 5,
wobei ein Immun-Kolloidgold-Teststreifen eines Doppel-Antikörper-Sandwich-Verfahrens hergestellt wird, wobei der Antikörper 1AA4 als Beschichtungs-Antikörper und der Antikörper AH9 als goldmarkierter Antikörper verwendet wird; oder
der Antikörper AH9 als Beschichtungs-Antikörper und der Antikörper 1AA4 als goldmarkierter Antikörper verwendet wird.

## Revendications

1. Anticorps anti-carbapénémase de type IMP, dans lequel l'anticorps anti-carbapénémase de type IMP est : un anticorps 1AA4 comprenant une région variable de chaîne légère et une région variable de chaîne lourde, dans lequel les régions de détermination complémentaire (CDRs) de la région variable de chaîne légère comprennent trois séquences représentées dans SEQ ID NO : 1, SEQ ID NO : 2, et SEQ ID NO : 3 ; les CDRs de la région variable de chaîne lourde comprend trois séquences représentées dans SEQ ID NO : 4, SEQ ID NO : 5, et SEQ ID NO : 6 ;
ou,
un anticorps AH9 comprenant une région variable de chaîne légère et une région variable de chaîne lourde, dans lequel les CDRs de la région variable de chaîne légère comprennent trois séquences représentées dans SEQ ID NO : 11, SEQ ID NO : 12, et SEQ ID NO : 13 ; les CDRs de la région variable de chaîne lourde comprend trois séquences représentées dans SEQ ID NO : 14, SEQ ID NO : 15, et SEQ ID NO : 16.

2. Anticorps anti-carbapénémase de type IMP selon la revendication 1, dans lequel l'anticorps 1AA4, la région variable de chaîne légère a une séquence d'acides aminés représentée dans SEQ ID NO : 7, et la région variable de chaîne lourde a une séquence d'acides aminés représentée dans SEQ ID NO : 9 ;
ou,
dans l'anticorps AH9, la région variable de chaîne légère a une séquence d'acides aminés représentée dans SEQ ID NO : 17, et la région variable de chaîne lourde a une séquence d'acides aminés représentée dans SEQ ID NO : 19.

3. Acide nucléique, comprenant une séquence nucléotidique codant pour l'anticorps anti-carbapénémase de type IMP selon la revendication 1 ou 2.

4. Acide nucléique selon la revendication 3, dans lequel une séquence nucléotidique de l'acide nucléique pour coder la région variable de chaîne légère de l'anticorps 1AA4 est représentée dans SEQ ID NO : 8, et une séquence nucléotidique de l'acide nucléique pour coder la région variable de chaîne lourde de l'anticorps 1AA4 est représentée dans SEQ ID NO : 10 ;
ou,
une séquence nucléotidique de l'acide nucléique pour coder la région variable de chaîne légère de l'anticorps AH9 est représentée dans SEQ ID NO : 18, et une séquence nucléotidique de l'acide nucléique pour coder la région variable de chaîne lourde de l'anticorps AH9 est représentée dans SEQ ID NO : 20.

5. Utilisation de l'anticorps anti-carbapénémase de type IMP selon la revendication 1 ou 2 dans la détection in vitro d'un antigène de carbapénémase de type IMP.

6. Utilisation de l'anticorps anti-carbapénémase de type IMP dans la détection d'un antigène de carbapénémase de type IMP selon la revendication 5,
dans lequel l'anticorps anti-carbapénémase de type IMP est utilisé dans un kit de diagnostic in vitro ou une puce microfluidique ; et le kit de diagnostic in vitro est un kit de dosage immunologique d'or colloïdal, un kit de chimioluminescence, un kit de dosage radio-immunologique, un kit de dosage d'immunoabsorption enzymatique (ELISA), ou un kit de dosage immunologique au fluor.

7. Utilisation de l'anticorps anti-carbapénémase de type IMP dans la détection d'un antigène de carbapénémase de type IMP selon la revendication 5,
dans lequel une bande de test d'or colloïdal immunitaire d'un procédé sandwich à double anticorps est préparée, dans lequel l'anticorps 1AA4 est utilisé en tant qu'anticorps de revêtement et l'anticorps AH9 est utilisé en tant qu'anticorps marqué à l'or ; ou
l'anticorps AH9 est utilisé en tant qu'anticorps de revêtement et l'anticorps 1AA4 est utilisé en tant qu'anticorps marqué à l'or.
